# EUROPÄISCHE PATENTANMELDUNG

(11) **EP 1 378 243 A1**
(43) Veröffentlichungstag der Anmeldung: **07.01.2004**
(21) Anmeldenummer: 03014958.7
(22) Anmeldetag: 01.07.2003
(51) Int. Cl.: A61K 35/56, A61P 29/00, A61P 19/02

(54) **Wirkstoffmischung umfassend eine Mischung aus einer Lipid- und einer Kohlenhydratfraktion der Grünlippenmuschel**

(30) Priorität: 01.07.2002 DE 20210182 U
(71) Anmelder: Bio-Innovation Development Limited, Christchurch 1 (NZ)
(72) Erfinder: Lingens,Johann Matthias, 79-83 Hereford Street, Christchurch 1 (NZ)
(74) Vertreter: Barz, Peter, Dr.

(57) **Zusammenfassung**

Es wird eine Wirkstoffmischung beschrieben, die eine Lipidfraktion der Grünlippenmuschel (perna canaliculus) und eine Kohlenhydratfraktion der Grünlippenmuschel umfasst.

## Beschreibung

Die Erfindung betrifft eine Wirkstoffmischung umfassend Bestandteile der Grünlippenmuschel (perna canaliculus), nämlich eine Mischung aus einer Lipid- und einer Kohlenhydratfraktion der Grünlippenmuschel, und ihre Verwendung.

Der Gehalt an Inhaltsstoffen der Grünlippenmuschel ist als Naturprodukt naturgemäß Schwankungen unterworfen. Angaben für gefriergetrocknete Muschelfleisch-Produkte liegen z.B. in folgenden Bereichen: 47 bis 62 Gew.-% Proteine, 5 bis 22 Gew.-% Kohlenhydrate, 8 bis 20 Gew.-% Lipide und daneben Mineralien und Vitamine. Der natürliche Gehalt an Omega-3-Fettsäuren in der Grünlippenmuschel (perna canaliculus) ist relativ gering und bei der Muschelernte Schwankungen unterworfen.

Die Grünlippenmuschel wird seit Jahrzehnten in der Behandlung von entzündlichen und degenerativen Beschwerden des Bewegungsapparats eingesetzt. Die antiinflammatorische Wirkung der Grünlippenmuschel beruht nach Erkenntnissen der Forschung u.a. auf einer modulatorischen Wirkung auf Entzündungsmediatoren in Form einer COX-2 Hemmung, wobei in der Literatur als primäre entzündungshemmende Komponente der Lipidgehalt der Grünlippenmuschel angesehen wird.

Übliche Anwendungsformen für eine entzündungshemmende Wirkung sind die Grünlippenmuschel als solche oder ein gefriergetrocknetes Pulver der ganzen Muschel. In US-A-6083536 und WO 96/05164 werden z.B. Verfahren zur Herstellung von Lipidfraktionen bzw. -extrakten aus der Grünlippenmuschel beschrieben. In der Patentanmeldung NZ 328489 wird ein Verfahren zur Herstellung von Kohlenhydratfraktionen aus der Grünlippenmuschel beschrieben.

Die Aufgabe bestand in der Bereitstellung eines Präparats aus der Grünlippenmuschel, das gegenüber den bekannten Produkten ein erhöhtes Wirkpotential bezüglich einer entzündungshemmenden Wirkung aufweist.

Die Aufgabe wurde gelöst durch eine Wirkstoffmischung umfassend eine Mischung aus einer Lipid- und einer Kohlenhydratfraktion der Grünlippenmuschel, die überraschenderweise gegenüber den Einzelfraktionen und Produkten aus dem ganzen Muschelfleisch bezüglich der entzündungshemmenden Wirkung eine synergistische Wirkung zeigt.

Mit der erfindungsgemäßen Wirkstoffmischung wird überraschenderweise erreicht, das bekannte Wirkpotential der Grünlippenmuschel beim Einsatz in der Behandlung von entzündlichen und degenerativen Erscheinungen insbesondere des Bewegungsapparats (rheumatische Beschwerden) beim Menschen oder bei Tieren zu verstärken. Dadurch wird es möglich, bezogen auf eine Einheitsmenge eine höhere Wirksamkeit als bei herkömmlichen Grünlippenmuschel-Produkten zu erreichen, was eine Volumenverringerung der Mischung bei gleicher Wirkung ermöglicht. Durch den Zusatz einer nicht aus der Grünlippenmuschel stammenden Komponente, die eine oder mehrere zusätzliche Omega-3-Fettsäuren enthält, wird die entzündungshemmende Wirkung überraschenderweise zusätzlich synergistisch verstärkt.

Die Wirkstoffmischung umfasst eine Lipidfraktion der Grünlippenmuschel (perna canaliculus) und eine Kohlenhydratfraktion der Grünlippenmuschel (perna canaliculus). Neben der Mischung aus diesen beiden Fraktionen enthält die Wirkstoffmischung vorzugsweise keine weiteren aus der Grünlippenmuschel stammende Komponenten, es können aber gegebenenfalls gewisse Mengen z.B. aus Produkten der ganzen Muschel zugesetzt werden. Sofern neben den genannten Fraktionen andere Produkte der Grünlippenmuschel enthalten sind, machen sie im allgemeinen nicht mehr als 90 Gew.-%, bevorzugter nicht mehr als 50 Gew.-% oder 20 Gew.-%, bezogen auf die Trockenmasse der in der Wirkstoffmischung enthaltenen Grünlippenmuschel-Produkte insgesamt, aus.

Unter den Fraktionen werden hier Materialien verstanden, die durch Trenntechniken aus der Grünlippenmuschel erhalten werden. Dabei umfasst die Lipidfraktion hauptsächlich angereicherte Lipide der Grünlippenmuschel. Je nach Trennverfahren kann die Lipidfraktion andere Bestandteile aus der Muschel oder aus dem Trennverfahren eingeführte Substanzen als Beimengungen enthalten. Die Kohlenhydratfraktion umfasst hauptsächlich angereicherte Kohlenhydrate der Grünlippenmuschel. Je nach Trennverfahren kann auch die Kohlenhydratfraktion andere Bestandteile aus der Muschel oder aus dem Trennverfahren eingeführte Substanzen als Beimengungen enthalten.

Dem Fachmann sind die Trennverfahren zur Isolierung dieser Fraktionen bekannt. Lipidfraktionen und Kohlenhydratfraktionen aus der Grünlippenmuschel sind im Handel erhältlich. Die Fraktionen werden manchmal auch als Extrakte bezeichnet. Beispiele für verwendbare Handelsprodukte sind Lyprinol® von Pharmalink für eine Lipidfraktion und Mussel Glycogen von Aroma New Zealand Ltd. für eine Kohlenhydratfraktion.

Als Trenntechniken zur Isolierung der Fraktionen kommen alle bekannten Verfahren in Betracht. Bei der Trennung der Fraktionen aus der Grünlippenmuschel werden vorzugsweise Extraktionsverfahren, Fällungsverfahren, enzymatische und chromatographische Verfahren oder Kombinationen davon eingesetzt. Vor allem wenn die Produkte ohne chromatographische Verfahren erhalten werden, werden die Fraktionen häufig als Lipidextrakte bzw. Kohlenhydratextrakte bezeichnet.

Als Ausgangsmaterial zur Herstellung der Fraktionen kann z.B. homogenisiertes frisches Muschelfleisch oder getrocknetes bzw. gefriergetrocknetes Muschelfleisch eingesetzt werden. Neben der Verwendung des ganzen Muschelfleisches kann in einer vorteilhaften Ausführungsform als Ausgangsprodukt auch nur ein Bestandteil davon verwendet werden, z.B. vorzugsweise die Gonaden der Grünlippenmuschel. Diese werden geeigneterweise aus dem frischen Muschelfleisch entnommen und wie vorstehend beschrieben vorbereitet.

Die Lipidfraktion kann aus der gegebenenfalls vorbereiteten Grünlippenmuschel oder deren Bestandteil z.B. durch Extraktion mit überkritischem CO₂ oder mit Ethanol gewonnen werden, wobei gegebenenfalls eine weitere Reinigung durch Chromatographie erfolgt (siehe z.B. US-A-6083536 oder WO 96/05164).

Die Kohlenhydratfraktion kann aus der gegebenenfalls vorbereiteten Grünlippenmuschel oder deren Bestandteil z.B. durch Phenolextraktion und Ethanolausfällung erhalten werden (siehe z.B. NZ 328489). Eine weitere Möglichkeit besteht in einer enzymatischen Isolierung der Kohlenhydrate (Extraktion). Weitere verwendbare Extraktionsmittel sind Ethanol oder überkritisches CO₂. Auch bei der Kohlenhydratfraktion kann eine chromatographische Reinigung zweckmäßig oder notwendig sein.

Die Fraktionen können eine Mischung verschiedener Substanzen oder Molekulargewichte der jeweiligen Stoffklasse (Lipid, Kohlenhydrat) enthalten. Natürlich können auch nicht vollständige oder (z.B. durch Chromatographie) weiter aufgetrennte Fraktionen mit weniger Komponenten von Lipid oder Kohlenhydrat aus der Grünlippenmuschel verwendet werden (d.h. die eingesetzten Fraktionen müssen nicht den Gesamtgehalt an Lipiden bzw. Kohlenhydraten der Grünlippenmuschel enthalten). Andererseits können natürlich auch getrennt isolierte Fraktionen derselben Stoffklasse (Lipid, Kohlenhydrat) vereint verwendet werden.

Der Anteil der Lipide in der Lipidfraktion ist gegenüber dem Lipidanteil im Ausgangsmaterial aus der Grünlippenmuschel (ganze Muschel oder Teile der Muschel), bezogen auf die Gewichtstrockenmasse, erhöht, im allgemeinen um mindestens das 2-fache, das 3-fache oder das 4-fache oder sogar mehr. Gewöhnlich sind in der Lipidfraktion z.B. mindestens 40 Gew.-%, bevorzugter mindestens 60 Gew.-% oder mindestens 80 Gew.-% Lipide enthalten (Trockenmasse).

Der Anteil der Kohlenhydrate in der Kohlenhydratfraktion ist gegenüber dem Kohlenhydratanteil im Ausgangsmaterial aus der Grünlippenmuschel (ganze Muschel oder Teile der Muschel), bezogen auf die Gewichtstrockenmasse, erhöht, im allgemeinen um mindestens das 1,5-fache, das 2-fache oder das 3-fache oder sogar mehr. Gewöhnlich sind in der Kohlenhydratfraktion z.B. mindestens 30 Gew.-%, bevorzugter mindestens 40 Gew.-% oder mindestens 50 oder 60 Gew.-% Kohlenhydrate enthalten (Trockenmasse, d.h. ohne Wasser oder Lösungsmittel).

In der Wirkstoffmischung kann das Gewichtsverhältnis von Lipidfraktion und Kohlenhydratfraktion in weiten Bereichen variieren. Das Gewichtsverhältnis beträgt aber im allgemeinen z.B. 1:20 bis 20:1, vorzugsweise 1:10 bis 10:1. In einer bevorzugten Ausführungsform liegt das Gewichtsverhältnis bei 3:1 bis 1:3, wobei ein etwa gleicher Anteil (1:1,3 bis 1,3:1) besonders bevorzugt ist. Die angegebenen Gewichtsanteile beziehen sich auf die reinen Lipid- bzw. Kohlenhydratanteile und lassen eventuelle Beimengungen in den Fraktionen unberücksichtigt.

Ein Vorteil der erfindungsgemäßen Wirkstoffmischung besteht darin, die Relation der wirksamen Bestandteile der Grünlippenmuschel zu variieren, so dass eine Konzentrierung der Wirkstoffe und je nach Anwendungszweck eine optimale Relation ausgewählt werden können. Überdies kann durch die Variation der Mengenverhältnisse eine erhöhte entzündungshemmende Wirksamkeit z.B. gegenüber Produkten aus der ganzen Muschel mit festgelegten natürlichem Verhältnis erzielt werden.

Die Wirkstoffmischung kann in einer vorteilhaften Ausführungsform ferner eine zusätzliche Omega-3-Fettsäuren enthaltende Komponente enthalten. Omega-3-Fettsäuren sind allgemein bekannt. Beispiele sind Eicosapentaensäure (EPA, 20:5) und Docosahexaensäure (DHA, 22:6). Die Komponente kann eine oder mehrere Omega-3-Fettsäuren enthalten. Die Komponente stammt nicht von der Grünlippenmuschel. Sie dient der Anreicherung des Konzentrats mit Omega-3-Fettsäuren. Da die Komponente vorzugsweise natürlichen Ursprungs ist, kann sie neben den Omega-3-Fettsäuren auch andere Bestandteile enthalten. Natürlich kann die Komponente aber auch nur aus einer oder mehreren Omega-3-Fettsäuren bestehen.

Die Komponente kann pflanzlichen oder bevorzugt maritimen Ursprungs sein. Beispiele für Komponenten pflanzlichen Ursprungs, die Omega-3-Fettsäuren enthalten, sind pflanzliche Öle wie Leinöl, Hanföl, Sojaöl, Flachsöl, Nachtkerzenöl, Rapsöl und Walnussöl oder Produkte (z.B. Extrakte) daraus. Fische und Meeresfrüchte oder allgemein maritime Lebewesen enthalten ebenfalls Omega-3-Fettsäuren, so dass daraus gewonnene Produkte ebenfalls verwendet werden können. Diese Komponenten maritimen Ursprungs sind bevorzugt. Es können auch Mischungen verschiedener Omega-3-Fettsäuren enthaltener Komponenten verwendet werden, z.B. Mischungen aus Komponenten pflanzlichen und maritimen Ursprungs.

Die die zusätzlichen Omega-3-Fettsäuren enthaltende Komponente kann in fester bzw. trockener oder fluider Form vorliegen, z.B. als Öl oder vorzugsweise als Pulver. Nach einem in der Technik bekannten Verfahren kann es sich bei der Komponente um mikroverkapselte Produkte handeln. Unter Mikroverkapselung versteht man im allgemeinen z.B. eine Ummantelung fester oder flüssiger Partikel. Das Beschichtungsmaterial kann z.B. aus natürlichen (z.B. Stärke) oder synthetischen Substanzen (z.B. modifizierter Stärke) und weiteren Additiven bestehen. Solche Produkte sind im Handel erhältlich.

Bevorzugt wird Fischöl oder ein Fischöl-Pulver, das durch Mikroverkapselung erhalten wird, als Omega-3-Fettsäuren enthaltende Komponente eingesetzt. Durch die Mikroverkapselung wird das Fischöl z.B. vor Oxidation geschützt. Natürlich umfasst die Mischung dann auch die in der Ummantelung enthaltenen Stoffe.

Sofern zusätzliche Omega-3-Fettsäuren zugegeben werden, kann das Verhältnis von Lipidfraktion und Kohlenhydratfraktion zu der zusätzlichen Komponente, die eine oder mehre Omega-3-Fettsäuren enthält, in weiten Bereichen variiert werden. Im allgemeinen wird ein solches Mischungsverhältnis gewählt, dass das Gewichtsverhältnis von dem gemeinsamen Gewicht von Lipidfraktion und Kohlenhydratfraktion der Grünlippenmuschel (Trockengewicht) zu den Omega-3-Fettsäuren, die in der nicht aus der Grünlippenmuschel stammenden Komponente enthalten sind, im Bereich von 100:1 bis 1:30 liegt. Ein bevorzugter Bereich für das Gewichtsverhältnis ist 3:1 bis 1:3.

Die Wirkstoffmischung kann weitere übliche Komponenten enthalten, z.B. Mineralstoffe, Vitamine, Spurenelemente, Nährstoffe, Antioxidationsmittel, Stabilisatoren und/oder je nach Darreichungsform übliche Hilfsstoffe. Gegebenfalls kann die Wirkstoffmischung auch in einem Lösungsmittel, wie Wasser, einer isotonen Kochsalzlösung oder Ethanol, aufgenommen werden, um Lösungen, Emulsionen oder Suspensionen zu erhalten.

Die Wirkstoffmischung kann eine Trockenmischung, eine flüssige Mischung oder eine Emulsion oder Suspension darstellen. Bevorzugt liegt die Mischung als Paste, Fluid, Trockenmischung bzw. Pulver vor. Bevorzugte Darreichungsformen sind Kapseln, z.B. Weichgelatine- oder Hartgelatinekapseln, Dragees und bei Veterinärtherapeutika oder beim diätetischen Einsatz auch als Pulver oder in flüssiger Form. Eine weitere vorteilhafte Anwendung ist z.B. in Form von Gels, Pasten, Salben und Cremes für die lokale Applikation, z.B. zum Einreiben.

## Patentansprüche

1. Wirkstoffmischung umfassend eine Lipidfraktion der Grünlippenmuschel (perna canaliculus) und eine Kohlenhydratfraktion der Grünlippenmuschel.

2. Wirkstoffmischung nach Anspruch 1, **dadurch gekennzeichnet, dass** sie ferner eine nicht aus der Grünlippenmuschel stammende Komponente, die eine oder mehrere zusätzliche Omega-3-Fettsäuren enthält, umfasst.

3. Wirkstoffmischung nach einem der Ansprüche 1 und 2, **dadurch gekennzeichnet, dass** die die zusätzlichen Omega-3-Fettsäuren enthaltende Komponente maritimen Ursprungs ist.

4. Wirkstoffmischung nach einem der Ansprüche 2 und 3, **dadurch gekennzeichnet, dass** die die zusätzlichen Omega-3-Fettsäuren enthaltende Komponente ein durch Mikroverkapselung erhaltenes Pulver ist.

5. Wirkstoffmischung nach einem der Ansprüche 1 bis 4, **dadurch gekennzeichnet, dass** mindestens eine der beiden Fraktionen aus den Gonaden der Grünlippenmuschel ist.

6. Wirkstoffmischung nach einem der Ansprüche 1 bis 5, **dadurch gekennzeichnet, dass** das Gewichtsverhältnis von Lipidextrakt zu Kohlenhydratextrat 1:20 bis 20:1 beträgt.

7. Wirkstoffmischung nach einem der Ansprüche 1 bis 6, **dadurch gekennzeichnet, dass** sie ein Fluid, eine Paste, ein Pulver oder eine Trockenmischung ist.

8. Wirkstoffmischung nach einem der Ansprüche 1 bis 7, **dadurch gekennzeichnet, dass** sie ferner Mineralstoffe, Vitamine, Spurenelemente, Nährstoffe, Antioxidationsmittel, Stabilisatoren und/oder je nach Darreichungsform übliche Hilfsstoffe umfasst.

9. Verwendung der Wirkstoffmischung nach einem der Ansprüche 1 bis 8 zur Behandlung von entzündlichen und degenerativen Krankheitszuständen.
